# EUROPEAN PATENT SPECIFICATION

(11) **EP 4 006 010 B1**
(45) Date of publication and mention of the grant of the patent: **11.10.2023**
(21) Application number: 20847360.3
(22) Date of filing: 05.06.2020
(51) Int. Cl.: C07C 253/34, C07C 255/46, C07C 57/145, C07C 253/30

(54) **IMPROVED METHOD FOR PREPARING MALEATE SALT OF PARA-SUBSTITUTED CIS-CYCLOHEXYLAMINO NITRILE**
VERBESSERTES VERFAHREN ZUR HERSTELLUNG VON MALEATSALZ VON PARA-SUBSTITUIERTEM CIS-CYCLOHEXYLAMINONITRIL
PROCÉDÉ AMÉLIORÉ DE PRÉPARATION DE SEL DE MALÉATE DE CIS-CYCLOHEXYLAMINONITRILE PARA-SUBSTITUÉ

(30) Priority: 26.07.2019 CN 201910685036
(43) Date of publication of application: 01.06.2022
(73) Proprietor: Hebei Lansheng Biotech Co., Ltd, Shijiazhuang, Hebei 052260 (CN); Hebei Lanrun Plant Protection Technology Co., Ltd., Cangzhou, Hebei 061100 (CN); Hebei Guzhirun Technology Co., Ltd, Shijiazhuang, Hebei 050000 (CN)
(72) Inventor: GUO, Qingchun, Shijiazhuang, Hebei 052260 (CN); DONG, Zhipeng, Shijiazhuang, Hebei 052260 (CN); YUAN, Ligang, Shijiazhuang, Hebei 052260 (CN); LIU, Hailong, Shijiazhuang, Hebei 052260 (CN); GUO, Hongyong, Shijiazhuang, Hebei 052260 (CN)
(74) Representative: karo IP
(86) International application number: PCT/CN2020/094606
(87) International publication number: WO 2021/017642

(56) References cited:
- EP-A1- 4 006 010
- EP-B1- 2 655 319
- WO-A1-00/35873
- CN-A- 103 270 020
- CN-A- 109 206 410
- CN-A- 110 294 692
- AHER S ET AL: "Ultrasound assisted cocrystallization from solution (USSC) containing a non-congruently soluble cocrystal component pair: Caffeine/maleic acid", EUROPEAN JOURNAL OF PHARMACEUTICAL SCIENCES, ELSEVIER AMSTERDAM, NL, vol. 41, no. 5, 23 December 2010 (2010-12-23), pages 597-602, XP027493534, ISSN: 0928-0987, DOI: 10.1016/J.EJPS.2010.08.012 [retrieved on 2010-08-27]
- Lan Shengyu; Huang Yongchun: "Research on Ultrasonic Enhanced Solution Crystallization", Guangxi Sugarcane & Cane sugar, no. 4, 31 December 2012 (2012-12-31), pages 23-27, XP009525758, ISSN: 1007-4732

## Description

This application claims the priority of the Chinese patent application No. 201910685036.0 filed on July 26, 2019 with the title of "an improved method for preparing cis-para-substituted cyclohexylaminonitrile maleate".

### Technical field

The present invention relates to an improved method for preparing cis-para-substituted cyclohexylaminonitrile maleate.

### Background technique

Maleic acid can separate the para-substituted cyclohexylaminonitrile compounds with good selectivity, and the separated cis-salt compound obtained from salt formation is an important intermediate for preparing spirotetramat.

However, since maleic acid is a solid which has low solubility in the reaction solvent, and the cis-para-substituted cyclohexylaminonitrile maleate obtained from salt formation also has low solubility, and the cis-isomer salt deposits on the surface of free maleic acid and coats it. The coated free maleic acid is blocked from forming salt, and eventually the unsalted maleic acid contaminates the product, which affects the quality of the product.

EP 2 655 319 describes several acid salts of para-substituted cyclohexylaminonitrile derivatives and relates to the preparation of acid salts of cis-para-substituted cyclohexylaminonitrile.

### Summary of the invention

The inventors of the present application found that in the preparation process of cis-para-substituted cyclohexylaminonitrile maleate, if ultrasonic vibration is performed while stirring, the interface effect of ultrasonic can prevent the maleate obtained from salt formation from depositing on the surface of free maleic acid solid particles and coating it, and further prevent the coated maleic acid from being difficult to further form salt, thereby making the salt-forming reaction more complete, and reducing the pollution of free maleic acid in the final product.

Furthermore, the inventors of the present application found that the water content in the salt-forming reaction system could affect the stability of the obtained maleate, and more stable product can be prepared in an anhydrous or low-moisture system with a moisture content of <_1%. However, there is the following contradiction: from the perspective of product stability, the above-mentioned salt-forming reaction is more advantageous in anhydrous or low-moisture system, but in anhydrous or low-moisture system with a moisture content of ≤1%, the above-mentioned coat problem is more serious since the maleate is more difficult to dissolve.

The present invention can solve the above contradiction through ultrasound assistance, not only can the salt-forming reaction proceed quickly and fully, but also a more stable product can the obtained. Therefore, it is more advantageous for the method of the present invention to be applied to the above-mentioned reaction system.

The invention of the present application was completed based on the above findings. Specifically, the present application provides an improved method for preparing cis-para-substituted cyclohexylaminonitrile maleate as shown in the following Formula (I).

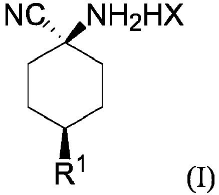

In Formula (I), R¹ is C₁₋₁₀ alkyl or alkyloxy, C₁₋₁₀ alkenyl or alkenyloxy, C₁₋₁₀ alkynyl or alkynyloxy, C₁₋₁₀ cycloalkyl or cycloalkyloxy, C₁₋₁₀ heterocycloalkyl or heterocycloalkyloxy containing 1-2 heteroatoms selected from O and N, and HX is maleic acid;

The method includes adding 0.3-0.55 mole times maleic acid to the organic solvent solution of the cis/trans para-substituted cyclohexyl aminonitrile compound corresponding to Formula (I) above, performing stirring and ultrasonic treatment, and then separating to obtain the cis-para-substituted cyclohexyl aminonitrile maleate as shown in Formula (I) above.

### Detailed description of the invention

The raw material compound used in the present invention, which is the cis/trans para-substituted cyclohexylaminonitrile compound corresponding to the above formula (I), can be synthesized by a known method disclosed in, for example, Chinese Patent Document CN103270020A.

In the above formula (I), R¹ is C₁₋₁₀ alkyl or alkyloxy, C₁₋₁₀ alkenyl or alkenyloxy, C₁₋₁₀ alkynyl or alkynyloxy, C₁₋₁₀ cycloalkyl or cycloalkyloxy, C₁₋₁₀ heterocycloalkyl or heterocycloalkyloxy containing 1-2 heteroatoms selected from O and N; R¹ is preferably the above groups with 1 to 6 carbon atoms, more preferably C₁₋₆ alkyl or alkyloxy, C₁₋₆ cycloalkyl or cycloalkyloxy, C₁₋₆ heterocycloalkyl or heterocycloalkyloxy containing 1-2 heteroatoms selected from O and N, further more preferably C₁₋₆ alkyl or alkyloxy, particularly preferably methyl or methoxy.

In the method of the present invention, ultrasonic vibration is performed while stirring, and the interface effect of ultrasonic is used to make the salt formed by the para-substituted cyclohexylaminonitrile compound and maleic acid difficult to accumulate on the surface of the undissolved and unsalted maleic acid solid surface and reduce deposition. Therefore, the free maleic acid fully forms a salt with the para-substituted cyclohexylaminonitrile compound at a faster rate. At the same time, it also reduces product contamination caused by coating the free maleic acid.

It can be expected that the method of the present invention can also be used in other reactions that have similar problems as mentioned above.

Ultrasound can be achieved by installing an ultrasonic device in the reaction equipment. The ultrasonic power can be adjusted according to the amount of materials, etc., preferably 0.1 to 0.8 w/L.

The time for stirring and ultrasonic treatment is usually 1 to 10 hours, preferably 2 to 8 hours, more preferably 3 to 6 hours.

The temperature is maintained at 5-40°C during stirring and ultrasonic treatment, preferably at 20-30°C, more preferably at 25-30°C.

In view of obtaining a more stable product, the organic solvent used in the preparation method of the present invention is preferably an anhydrous organic solvent or a low-moisture organic solvent with moisture content of ≤1%, more preferably toluene or xylene.

The amount of maleic acid used is preferably 0.3-0.55 mole times, more preferably 0.3-0.5 mole times relative to the cis/trans para-substituted cyclohexylaminonitrile compound.

After stirring and ultrasonic treatment, the cis-para-substituted cyclohexylaminonitrile maleate is separated by filtration or the like.

### Examples

Hereinafter, the present invention will be explained more specifically through examples, but the present invention is not limited to these examples.

### Example 1

Added 181kg (0.4mol times) maleic acid to a toluene solution(the total solution was 1500kg) of 602kg p-methoxycyclohexylaminonitrile (cis: trans=55:45), and maintained the temperature at 25 ~ 30°C after the addition, performed stirring and ultrasonic vibration for 2 hours, the ultrasonic power was 0.4 w/L. Then the precipitated maleate was filtered, rinsed with appropriate amount of toluene, and dried to obtain white cis-p-methoxycyclohexyl aminonitrile maleate (386 kg, cis:trans= 95.5:4.5).

The obtained product was determined by a gas phase internal standard method with p-xylene as the internal standard under the following conditions to determine the content of cyanamide maleate, and the amount of maleic acid combined with cyanamide was converted from the above content. The amount of maleic acid fed minus the amount of maleic acid combined with cyanamide is the amount of coated free maleic acid.
Gas phase conditions: FID detector
Injection port: 245°C
Detector: 250°C
Program temperature rise: kept the initial temperature at 60°C for 0 min, raised the temperature to 110°C at 11.5°C/min, held for 2.5min, raised the temperature to 200°C at 11.5°C/min, held for Imin.
Split ratio:10:1, injection volume: 0.2ul, flow rate: 8.0ml/min
Chromatographic column: SE-54 medium polarity chromatographic column 30m×0.53mm

The measurement result of this example was that the coated free maleic acid accounted for 8.5% of the total maleic acid fed.

Stored 20g of the obtained cis-p-methoxycyclohexylaminonitrile maleate in a sample room at 25-30°C, 40-45% humidity. The decomposition rate was 0.34% on the 7th day.

### Example 2

The time for performing stirring and ultrasonic vibration was adjusted to 4 hours, and the others were the same as Example 1, 404.5kg white cis-p-methoxycyclohexylaminonitrile maleate was obtained (cis:trans =96.8:3.2).

Measured by the same method as Example 1, the coated free maleic acid accounted for 4.1% of the total maleic acid fed, and the decomposition rate of the obtained cis-p-methoxycyclohexylaminonitrile maleate on the 7th day was 0.37%.

### Comparative example 1

Added 181kg (0.4mol times) maleic acid to a toluene solution(the total solution is 1500kg) of 602kg p-methoxycyclohexylaminonitrile (cis: trans=55:45), and maintained the temperature at 25 ~ 30°C after the addition, and stirred for 12 hours, then filtered the precipitated maleate, rinsed with appropriate amount of toluene, and dried to obtain white cis-p-methoxy cyclohexylaminonitrile maleate (380.5kg, cis:trans=92.7:7.3).

Measured by the same method as Example 1, the coated free maleic acid accounted for 9.8% of the total maleic acid fed, and the decomposition rate of the obtained cis-p-methoxycyclohexylaminonitrile maleate on the 7th day was 0.41%.

### Comparative example 2

Ethyl acetate was used instead of toluene as the solvent, and the other operations are the same as Comparative Example 1.

Measured by the same method as Example 1, the coated free maleic acid accounted for 11% of the total maleic acid fed, and the decomposition rate of the obtained cis-p-methoxycyclohexylaminonitrile maleate on the 7th day was 7.90%.

It can be known from the results of the above examples and comparative examples that without ultrasound assistance, even if the reaction time was prolonged by 3 or 6 times, the percentage of free maleic acid that was finally coated and unsalted was higher. At the same time, the products made with low-moisture solvents such as toluene have higher stability.

### Industrial applicability

The method of the present invention can avoid free maleic acid coating problem in the preparation process of the cis-p-methoxy cyclohexylaminonitrile maleate by introducing ultrasonic assistance, and obtain a product with better quality. Using this product, spirotetramat with better quality can be prepared.

## Claims

1. An improved method for preparing the cis-para-substituted cyclohexylaminonitrile maleate represented by Formula (I): 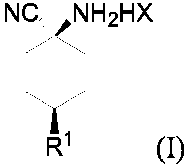
In the above Formula (I), R¹ is C₁₋₁₀ alkyl or alkyloxy, C₁₋₁₀ alkenyl or alkenyloxy, C₁₋₁₀ alkynyl or alkynyloxy, C₁₋₁₀ cycloalkyl or cycloalkyloxy, C₁₋₁₀ heterocycloalkyl or heterocycloalkyloxy containing 1-2 heteroatoms selected from O and N, HX is maleic acid.
The method includes adding 0.3-0.55 mole times maleic acid to the organic solvent solution of the cis/trans para-substituted cyclohexyl aminonitrile compound corresponding to Formula (I) above, performing stirring and ultrasonic treatment, then separating to obtain the cis-para-substituted cyclohexyl aminonitrile maleate as shown in Formula (I) above.

2. The method according to claim 1, wherein R¹ is C₁₋₆ alkyl or alkyloxy, C₁₋₆ alkenyl or alkenyloxy, C₁₋₆ alkynyl or alkynyloxy, C₁₋₆ cycloalkyl or cycloalkyloxy, C₁₋₆ heterocycloalkyl or heterocycloalkyloxy containing 1-2 heteroatoms selected from O and N.

3. The method according to claim 1, wherein R¹ is C₁₋₆ alkyl or alkyloxy, preferably methyl or methoxy.

4. The method according to claim 1, wherein the temperature is maintained at 5-40°Cduring stirring and ultrasonic treatment, preferably at 20-30°C, more preferably at 25-30°C.

5. The method according to claim 1 above, wherein the organic solvent is an anhydrous organic solvent or a low-moisture organic solvent with a moisture content of < 1 %.

6. The method according to claim 1, wherein the organic solvent is toluene or xylene.

7. The method according to claim 1, wherein the time for stirring and ultrasonic treatment is 1-10 hours, preferably 2-8 hours, more preferably 3-6 hours.

8. The method according to claim 1, wherein the ultrasonic power is 0.1-0.8 w/l.

9. The method according to claim 1, wherein the added amount of maleic acid is 0.3-0.5 mole times.

10. The method according to claim 1, wherein the separation is conducted by filtration.

## Patentansprüche

1. Ein verbessertes Verfahren zur Herstellung von cis-para-substituiertem Cyclohexylaminonitrilmaleat, dargestellt in Formel (I): 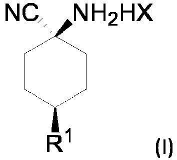
In obiger Formel (I) ist R¹ ein C₁₋₁₀-Alkyl- oder -Alkyloxyrest, ein C₁₋₁₀-Alkenyl- oder - Alkenyloxyrest, ein C₁₋₁₀-Alkinyl- oder -Alkinyloxyrest, ein C₁₋₁₀-Cycloalkyl- oder - Cycloalkyloxyrest, ein C₁₋₁₀-Heterocycloalkyl- oder-Heterocycloalkyloxyrest, der 1-2 Heteroatome ausgewählt aus O und N enthält, und HX ist Maleinsäure.
Das Verfahren umfasst die Zugabe von 0,3-0,55 Mol Maleinsäure zu der organischen Lösungsmittellösung der cis/trans-para-substituierten Cyclohexylaminonitrilverbindung, die der obigen Formel (I) entspricht, die Anwendung von Rühren und Ultraschallbehandlung und die anschließende Trennung, um das cis-para-substituierte Cyclohexylaminonitrilmaleat nach Formel (I) zu erhalten.

2. Verfahren nach Anspruch 1, wobei R¹ C₁₋₆-Alkyl oder -Alkyloxy, C₁₋₆-Alkenyl oder - Alkenyloxy, C₁₋₆-Alkinyl oder -Alkinyloxy, C₁₋₆-Cycloalkyl oder -Cycloalkyloxy, C₁₋₆-Heterocycloalkyl oder -Heterocycloalkyloxy mit 1-2 Heteroatomen, ausgewählt aus O und N, ist.

3. Verfahren nach Anspruch 1, wobei R¹ C₁₋₆-Alkyl oder Alkyloxy, vorzugsweise Methyl oder Methoxy ist.

4. Verfahren nach Anspruch 1, wobei die Temperatur während des Rührens und der Ultraschallbehandlung bei 5-40°C, bevorzugt bei 20-30°C, mehr bevorzugt bei 25-30°C gehalten wird.

5. Verfahren nach Anspruch 1, wobei das organische Lösungsmittel ein wasserfreies organisches Lösungsmittel oder ein organisches Lösungsmittel mit einem geringen Feuchtigkeitsgehalt von ≤ 1% ist.

6. Verfahren nach Anspruch 1, wobei das organische Lösungsmittel Toluol oder Xylol ist.

7. Verfahren nach Anspruch 1, wobei die Zeit für das Rühren und die Ultraschallbehandlung 1-10 Stunden, bevorzugt 2-8 Stunden, mehr bevorzugt 3-6 Stunden beträgt.

8. Verfahren nach Anspruch 1, wobei die Ultraschallenergie 0,1-0,8 W/I beträgt.

9. Verfahren nach Anspruch 1, wobei die zugesetzte Menge an Maleinsäure das 0,3-0,5fache Mol beträgt.

10. Verfahren nach Anspruch 1, wobei die Trennung durch Filtration durchgeführt wird.

## Revendications

1. Procédé amélioré de préparation du maléate de cyclohexylaminonitrile cis-para-substitué représenté par la formule (I) : 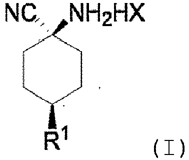
dans la formule (I) ci-dessus, R¹ est alkyle ou alkyloxy en C₁₋₁₀, alcényle ou alcényloxy en C₁₋₁₀, alcynyle ou alcynyloxy en C₁₋₁₀, cycloalkyle ou cycloalkyloxy en C₁₋₁₀, hétérocycloalkyle ou hétérocycloalkyloxy en C₁₋₁₀ contenant 1 à 2 hétéroatomes choisis parmi O et N, HX est l'acide maléique,
le procédé comprend l'ajout de 0,3 à 0,55 fois en moles d'acide maléique à la solution dans un solvant organique du composé cyclohexylaminonitrile cis/trans para-substitué correspondant à la formule (I) ci-dessus, la conduite d'un traitement par agitation et ultrasons, puis une séparation pour obtenir le maléate de cyclohexylaminonitrile cis-para-substitué tel que décrit dans la formule (I) ci-dessus.

2. Procédé selon la revendication 1, dans lequel R¹ est alkyle ou alkyloxy en C₁₋₆, alcényle ou alcényloxy en C₁₋₆, alcynyle ou alcynyloxy en C₁₋₆, cycloalkyle ou cycloalkyloxy en C₁₋₆, hétérocycloalkyle ou hétérocycloalkyloxy en C₁₋₆ contenant 1 à 2 hétéroatomes choisis parmi O et N.

3. Procédé selon la revendication 1, dans lequel R¹ est alkyle ou alkyloxy en C₁₋₆, de préférence méthyle ou méthoxy.

4. Procédé selon la revendication 1, dans lequel la température est maintenue à 5 à 40 °C pendant le traitement par agitation et ultrasons, de préférence à 20 à 30 °C, plus préférablement à 25 à 30 °C.

5. Procédé selon la revendication 1 ci-dessus, dans lequel le solvant organique est un solvant organique anhydre ou un solvant organique à faible humidité avec une teneur en humidité ≤ 1 %.

6. Procédé selon la revendication 1, dans lequel le solvant organique est le toluène ou le xylène.

7. Procédé selon la revendication 1, dans lequel la durée du traitement par agitation et ultrasons est de 1 à 10 heures, de préférence 2 à 8 heures, plus préférablement 3 à 6 heures.

8. Procédé selon la revendication 1, dans lequel la puissance ultrasonore est de 0,1 à 0,8 W/I.

9. Procédé selon la revendication 1, dans lequel la quantité d'acide maléique ajoutée est de 0,3 à 0,5 fois en moles.

10. Procédé selon la revendication 1, dans lequel la séparation est conduite par filtration.
